# EUROPÄISCHE PATENTSCHRIFT

(11) **EP 3 630 009 B1**
(45) Veröffentlichungstag und Bekanntmachung des Hinweises auf die Patenterteilung: **27.11.2024**
(21) Anmeldenummer: 18729901.1
(22) Anmeldetag: 30.05.2018
(51) Int. Cl.: A61F 2/07, A61F 2/06, A61F 2/89

(54) **STENTGRAFT MIT TASCHEN**
STENT GRAFT WITH POCKETS
ENDOPROTHÈSE À POCHES

(30) Priorität: 31.05.2017 DE 102017111964
(43) Veröffentlichungstag der Anmeldung: 08.04.2020
(73) Patentinhaber: JOTEC GmbH, 72379 Hechingen (DE)
(72) Erfinder: DERKVIST, Stefan, 72379 Hechingen (DE)
(74) Vertreter: Witte, Weller & Partner Patentanwälte mbB
(86) Internationale Anmeldenummer: PCT/EP2018/064233
(87) Internationale Veröffentlichungsnummer: WO 2018/220043

(56) Entgegenhaltungen:
- WO-A1-2015/126768
- WO-A1-2016/183128
- US-A- 5 123 917
- US-A- 5 843 166
- US-A1- 2007 219 622

## Beschreibung

Die vorliegende Erfindung betrifft ein Stentgraft zur Implantation in Gefäße eines Patienten, mit einem aus einem Prothesenmaterial bestehenden hohlzylindrischen Grundkörper und mit zumindest einem den Grundkörper umlaufenden Stent-Element.

Derartige Stentgrafts sind im Stand der Technik beispielsweise durch DE 103 37 739 hinreichend bekannt. Es handelt sich hierbei um Gefäßprothesen, die auch als endovaskuläre Gefäßstents/-stentgrafts bezeichnet werden, welche allgemein zur Unterstützung von labilen, brüchigen bzw. thrombotischen Gefäßwänden und insbesondere zur Behandlung von aneurysmatischen Gefäßen eingesetzt werden. Hierfür wird an der erkrankten oder verletzten Stelle des Gefäßes ein Stentgraft freigesetzt, der die Funktionalität des ursprünglichen Gefäßes wieder herstellt bzw. die noch bestehende Gefäßintegrität unterstützt.

Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet. Ursache eines thorakalen und thorakoabdominalen Aortenaneurysmas können Arteriosklerose, Bluthochdruck sowie Entzündungsprozesse der Gefäßwand sein. Auch Verletzungen des Brustkorbes durch schwere Unfälle können akut oder chronisch zu einem Aortenaneurysma führen.

Je nach Anwendungsart werden die unterschiedlichsten Stentgrafts eingesetzt. Hierbei wird zwischen ballonexpandierbaren und selbstexpandierenden Systemen unterschieden. Die selbstexpapandierbaren Eigenschaften ergeben sich aus der Verwendung von selbstexpandierenden Materialien, wie beispielsweise Nitinol. Ballonexpandiebare Systeme werden durch eine von innen ausgeübte radiale Kraft expandiert, wenn sie beispielsweise auf einem Ballon montiert sind.

Die hier vorliegende Anmeldung beschäftigt sich mit dem Applizieren von sogenannten selbstexpandierenden Stentgrafts bzw. "gecoverten" Stents.

Zur Behandlung von Aneurysmen bzw. von zu verschließen drohenden Gefäßen ist es bereits bekannt, die betroffenen Blutgefäße durch Implantation eines Stents/Stentgrafts zu stabilisieren, um eine Ruptur des Gefäßes zu vermeiden bzw. allgemein die Offenhaltung des Gefäßes zu gewährleisten. Die verwendeten selbstexpandierende Stentgrafts bestehen im Allgemeinen aus einem hohlzylindrischen Metallrahmen bzw. -gerüst, dessen Mantelfläche mit einer Textil- oder Polymerfolie abgedeckt ist, so dass sich ein hohlzylindrischer Körper ergibt. Die Bioverträglichkeit der verwendeten Materialien führt dazu, dass der Kontakt zwischen dem expandierten Stentgraft und der Gefäßwand komplikationsfrei ist.

Eine weitere Funktion der Textil- bzw. Polymerfolie besteht in der Verhinderung eines Blutdurchtritt bzw. eines Durchtritts von Blutbestandteilen oder Ablagerungen durch die Wandung des Stentgrafts, sowie eins Einwachsens von Gewebe durch die Wandung ins Innere des Stentgrafts. Damit wird die Gefäßwand an der Implantationsstelle des Stentgrafts entlastet und mögliche Embolien an diesen Stellen werden verhindert.

Der Metallrahmen solcher Stentgrafts besteht in der Regel bspw. aus einem Drahtgeflecht oder aus hintereinander angeordneten, mäanderförmig umlaufenden, sogenannten Stentfedern, die ggf. über Verbindungsstützen aus Draht miteinander verbunden sind, oder die lediglich über das Prothesenmaterial miteinander verbunden sind. Das Drahtgeflecht bzw. die Stentfedern sind üblicherweise aus einem Shape-Memory-Material, in der Regel aus Nitinol, wodurch die Stentfedern nach Einbringung in ein Gefäß zur Freisetzung wieder in den expandierten Zustand übergehen und der Stentgraft dadurch "aufspannt".

Bei der Implantation des Stents wird dieser radial zusammengedrückt, so dass sich seine Querschnittsfläche deutlich verringert. Um den komprimierten Zustand herzustellen, wird der Stentgraft in eine Hülle eingebracht, die als Hüllkatheter bezeichnet wird. Der Hüllkatheter weist dazu einen Umfang auf, der kleiner ist als der Umfang des Stentgrafts im expandierten Zustand. Der Stentgraft wird im komprimierten Zustand mit Hilfe eines Einführsystems in den Bereich der zu behandelnden Stelle bzw. des Aneurysmas gebracht, wo er freigesetzt wird, wobei die Position des Stentgrafts über Röntgenmarker kontrolliert werden kann. Nachdem die gewünschte Position bzw. Ausrichtung des Stentgraftes erreicht ist, wird der Hüllkatheter zurückgezogen, wodurch der Stentgraft freigesetzt wird und expandieren kann. Auf Grund der Federwirkung des Metallrahmens/- gerüsts expandiert der Stentgraft wieder in seine ursprüngliche Form und spannt dabei seine Mantelfläche auf, die sich proximal und distal von dem Aneurysma bzw. der offen zu haltenden Stelle innen in dem Blutgefäß verklemmt. Durch die Druckkraft zwischen dem Stentgraft und der Gefäßwand bleibt der Stentgraft an der gewünschten Position lagefest. Auf diese Weise fließt das Blut jetzt durch den Stentgraft, wobei eine weitere Belastung der Aussackung verhindert, sowie die Offenhaltung des Gefäßes gewährleistet wird.

Bei der Positionierung der Stentgrafts dürfen die vom Hauptgefäß abgehenden seitlichen Blutgefäße durch das blutdichte Prothesenmaterial nicht von der Blutzufuhr abgeschnitten werden. Daher weisen viele Stentgrafts offene Zonen oder sogenannte Fenestrierungen im Mantel- bzw. Prothesenmaterial auf, über welche vom Stentgraft abgehende Seitenäste, die in die Seitengefäße hineinragen, eingeführt und am Stentgraft fixiert werden können. Dadurch wird auch eine Blutversorgung der Körperbereiche, die von den seitlichen Gefäßen versorgt werden, gewährleistet.

Aufgrund der Tatsache, dass die Beschaffenheit sowohl von den zu behandelnden Gefäßen, als auch von dem jeweiligen Patienten abhängig ist, ist es durchaus sinnvoll maßangefertigte Stentgrafts bezüglich der Anatomie des speziellen Gefäßes herzustellen. Dies ist jedoch nicht nur hinsichtlich der individuellen Ausgestaltung des Maßstabs, bezüglich der Länge und Breite aufwendig, sondern vielmehr ist auch die eigentliche Fertigung der Stents sehr aufwendig, teuer sowie arbeits- und kostenintensiv, da in bestimmten Fällen jeweils nur eine spezifische Prothese gefertigt werden kann.

Bei der Herstellung von Stentgrafts, insbesondere derjenigen, die mäanderförmig umlaufende Stentfedern aufweisen und lediglich über das Implantatmaterial und damit indirekt miteinander verbunden sind, werden die Mantelflächen an den Metallrahmen/ -gerüst händisch durch Fachkräfte vernäht, was durchaus sehr zeit- und kostenintensiv ist.

Die US 2007/0219622 A1, die WO 2016/183128 A1, die US 5,123,917 und die US 5,843,166 offenbaren jeweils einen Stentgraft, der aus zwei übereinander angeordneten Prothesenmaterial-Schläuchen besteht, mittels welchen Taschen gebildet werden.

Nach wie vor besteht daher ein großes Bedürfnis nach Stent-/ Stentgraftsystemen, bzw. Gefäßprothesen, mit Hilfe derer ein flexibler Einsatz ermöglicht wird, um den unterschiedlichen Anforderungen - insbesondere im Hinblick auf die individuelle Ausgestaltung - der jeweiligen Gefäße verschiedener Patienten nachzukommen.

Aufgabe der vorliegenden Erfindung ist es daher, einen alternativen Stentgraft bereitzustellen, mit welcher die oben geschilderten Nachteile der im Stand der Technik bekannten Stentgrafts überwunden werden können, und mit welcher die Herstellung weniger zeit- und kostenintensiv ist.

Erfindungsgemäß wird die vorherstehend genannte und andere Aufgaben gelöst durch einen Stentgraft zur Implantation in Gefäße, insbesondere Blutgefäße, eines Patienten nach Anspruch 1, wobei der Stentgraft folgendes aufweist: einen aus einem ersten Prothesenmaterial bestehenden hohlzylindrischen Grundkörper, mit einem proximalen und einem distalen Ende, mit einer Längsachse c und einem Umfang u, zumindest ein aus einem zweiten Prothesenmaterial bestehendes Taschenelement, das umlaufend auf der Außen- und/oder Innenseite des Grundkörpers zur Ausbildung einer umlaufenden geschlossenen Tasche auf einem Längsabschnitt des Grundkörpers angebracht ist, und mindestens ein den Grundkörper mäanderförmig umlaufendes Stent-Element, das innerhalb des Taschenelements aufgenommen ist, wobei das zumindest eine Taschenelement kürzer als der Grundkörper ist, und ein proximales und ein distales Ende sowie einen dazwischen liegenden Hauptabschnitt aufweist, wobei das zumindest eine Taschenelement zur Ausbildung der geschlossenen Tasche lediglich über sein jeweils distales und proximales Ende umfänglich außen und/oder innen am Grundkörper befestigt und der Hauptabschnitt nicht am Grundkörper befestigt ist, und wobei zwischen 2, 3, 4, 5, 6, 7, 8, 9, 10, 11, 12, 13, 14, 15, 16, 17, 18, 19, 20, 25 oder 30 Taschenelemente vorgesehen sind, die in einem Abstand x und getrennt voneinander auf dem Grundkörper fixiert sind.

Die Erfindung zugrunde liegende Aufgabe wird dadurch vollkommen gelöst.

Mit dem erfindungsgemäßen Stentgraft wird eine Gefäßprothese bereitgestellt, welche zur Unterstützung von labilen, brüchigen bzw. thrombischen Gefäßwänden und insbesondere zur Behandlung von aneurysmatischen Gefäßen eingesetzt werden kann. Dies wird durch den speziellen Aufbau des Stentgrafts erreicht, der auf einem hohlzylindrischen Grundkörper aus einem ersten Prothesenmaterial sowie Stent-Elementen basiert, wobei die Stent-Elemente nicht direkt - bspw. über Nähte - am Grundkörper fixiert sind, sondern in außen oder innen auf dem Grundkörper vorgesehenen geschlossenen Taschenelementen und damit in diesen praktisch "frei", d.h. nicht durch Nähte fixiert, vorliegen. Die Stent-Elemente sind also lediglich "indirekt" (= nicht durch Nähte fixiert) durch deren Aufnahme in die Taschenelemente am Grundkörper angebracht.

Gemäß einer bevorzugten Ausführungsform handelt es sich bei dem erfindungsgemäßen Stentgraft um einen selbstexpandierenden Stentgraft.

Der erfindungsgemäße Stentgraft hat den Vorteil, dass durch das Vorsehen von Taschenelementen und die einfache Aufnahme der Stent-Elemente innerhalb der Taschenelemente die einzelnen Stent-Elemente nicht aufwändig und jeweils einzeln mit vielen Nähten am Grundkörper fixiert werden müssen. Dadurch wird die Herstellung deutlich vereinfacht, wobei die grundsätzliche technische Funktion des Stentgrafts bzw. der Stent-Elemente, beibehalten wird. Somit ist die Herstellung weniger zeit- und kostenintensiv. Durch den speziellen Aufbau des erfindungsgemäßen Stentgraft, kann beispielsweise das händische Annähen der Stent-Elemente ersetzt werden durch maschinell gefertigte Nähte, welche die Taschen ausbilden, in denen sich die Stent-Elemente befinden. Gemäß einer alternativen Ausführungsform können die Taschen auch geklebt oder im Prothesenmaterial verschweißt werden.

Die Taschenelemente, in denen sich die Stent-Elemente befinden, können sowohl innenliegend als auch außenliegend in Bezug auf den hohlzylindrischen Grundkörper angebracht sein. Das bedeutet, dass der Grundkörper einen äußeren Mantel bildet, sofern die Taschenelemente innen liegen. Im umgekehrten Fall bildet der Grundkörper einen inneren Mantel, sofern die Taschenelemente außen liegen. In einer weiteren Ausführungsform kann es von Vorteil sein, die Taschenelemente sowohl innen- als auch außenliegend anzubringen.

Die einfache Herstellungsmöglichkeit des erfindungsgemäßen Stentgrafts ermöglicht es nicht nur, individuelle, auf einzelne zu behandelnde Patienten angepasste Stentgrafts anzufertigen, sondern andererseits auch, universell einsetzbare Stentgrafts vorzufertigen.

Die Stent-Elemente dienen erfindungsgemäß dem Zweck nicht nur dem Stentgraft die nötige Struktur zu geben, sondern im implantierten Zustand den Stentgraft an die Gefäßwand zu drücken und den Stentgraft so im Gefäß in Position zu halten. Die Eigenschaften der eingesetzten Stent-Elemente sind abhängig von dem zu behandelnden Gefäß. So ist beispielsweise bei einem sehr dünnwandigen Gefäß von Vorteil Stentgrafts, die weniger steife und in Summe weniger Stent-Elemente vorweisen, zu verwenden.

Aus dem speziellen Aufbau des erfindungsgemäßen Stentgrafts ergibt sich zudem der Vorteil, dass sowohl außen als auch innen nur das Prothesenmaterial vorliegt, welches bevorzugt biokompatibel ist.

Aufgrund dass die Stent-Elemente in geschlossenen Taschenelementen aufgenommen sind, wird auch ein mechanisches Verletzen oder Reizen der Gefäßwände durch im anderen Fall freiliegende Stent-Elemente vermieden.

Vorliegend ist mit "Grundkörper" der Hauptkörper des hohlzylindrischen Stentgrafts gemeint, welcher aus einem ersten Prothesenmaterial besteht.

Erfindungsgemäß ist das zumindest eine Taschenelement kürzer als der Grundkörper, und weist ein proximales und ein distales Ende sowie einen dazwischen liegenden Hauptabschnitt auf, wobei das zumindest eine Taschenelement zur Ausbildung der geschlossenen Tasche lediglich über sein jeweils distales und proximales Ende umfänglich außen und/oder innen am Grundkörper befestigt und der Hauptabschnitt nicht am Grundkörper befestigt ist.

Erfindungsgemäß befindet sich zumindest ein Taschenelement am Grundkörper, dessen Länge vorzugsweise an die Maße des jeweils einzubringenden Stent-Elements angepasst ist.

Erfindungsgemäß sind zwischen 1 und 30 Taschenelement vorgesehen, die in einem Abstand und getrennt voneinander auf dem Grundkörper fixiert sind.

Vorzugsweise weist der erfindungsgemäße Stentgraft zwischen 3 und 25, noch bevorzugter zwischen 5 und 20 Taschenelemente auf. Demnach sind 2, 3, 4, 5, 6, 7, 8, 9, 10, 11, 12, 13, 14, 15, 16, 17, 18, 19, 20, 25 oder 30 Taschenelemente für die entsprechende Anzahl an Stent-Elementen vorgesehen, die über die Längsachse des Grundkörpers verteilt und in einem Abstand voneinander angebracht sind. Die Länge der Taschenelemente - bezogen auf die Längsachse - ist immer kürzer als die Länge des Grundkörpers und den Maßen der Stent-Elemente angepasst.

Es ist auch denkbar unterschiedlich lange Taschenelemente vorzusehen, die unterschiedliche Stent-Elemente aufnehmen.

In dem Abschnitt/den Abschnitten, auf dem sich die Taschenelemente befinden, liegen dem Aufbau nach zwei Prothesenmaterialschichten übereinander, wobei eine Schicht das Prothesenmaterial des Grundkörpers ist und die andere Schicht das Prothesenmaterial des Taschenelements ist. Das Taschenelement wird dabei an seinen distalen als auch proximalen Enden umfänglich am Grundkörper befestigt.

Unter "mäanderförmig" wird vorliegend jeder schlingen- bzw. schleifenförmige Verlauf eines Stent-Elements verstanden.

Grundsätzlich werden bei Stentgrafts zur Bezeichnung der jeweiligen Enden die Begriffe "distal" und "proximal" verwendet, wobei der Begriff "distal" der Teil, bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt ist.

Erfindungsgemäß bedeutet die Einteilung des Grundkörpers des selbstexpandierende Stentgraft in proximaler Endabschnitt und distaler Endabschnitt, dass die jeweiligen Abschnitte durch eine voneinander unterschiedlichen Bauweise unterscheiden können.

So kann beispielsweise eine unterschiedliche Anzahl an Stent-Elementen und damit auch Taschenelementen in den Abschnitten des Grundkörpers vorliegen; anders gesprochen kann in einer Ausführungsform vorgesehen sein, dass bei vorliegen mehrerer Taschenelemente und Stent-Elemente diese - also die Taschenelemente - nicht gleichmäßig über die Längsachse des Grundkörpers verteilt sind. Auch können die in die Taschenelemente aufgenommenen Stent-Elemente unterschiedliche Durchmesser aufweisen, was zu unterschiedlichen Durchmessern des Grundköpers führen kann.

Vorliegend wird unter "Stent" bzw. "Stent- Element" jede Struktur bezeichnet, die einer Gefäßprothese eine Expansionskraft und/oder eine stützende Funktion verleiht. Entsprechend ist ein Stent-Element daher jedes Element, das die Eigenschaften eines Stent aufweist.

Der Ausdruck "Stentgraft" soll vorliegend - wie auch im Stand der Technik- eine Gefäßprothese bedeuten, die einen bzw. mehrere Stent-Elemente (oder Stentfedern) sowie ein damit indirekt oder direkt verbundenes Prothesen("graft")-Material zur Ausbildung eines Lumens durch zumindest einen Abschnitt des Stentgrafts bilden. Ein solcher Stentgraft wird auch als gecoverter Stent bezeichnet.

Die Taschenelemente, bzw. deren am Grundkörper fixierte Enden, sind gemäß einer Ausführungsform untereinander nicht direkt miteinander verbunden.

Gemäß einer anderen Ausführungsform sind die Enden der TaschenElemente direkt anliegend an den Enden eines proximal oder distal nachgeordneten Taschenelements vorgesehen.

Je nach Anwendungsbereich ist es vorteilhaft, wenn der erfindungsgemäße Stentgraft mehrere Taschenelemente und darin vorliegende Stent-Elemente aufweist. Die Anzahl der Stent-Elemente wird sich u.a. durch die Länge des benötigten Stentgrafts ergeben. Da die Länge des Stentgrafts bevorzugt zwischen 5 und 300 cm, vorzugsweise zwischen 8 und 26 cm, liegt, ergibt sich eine bevorzugte Anzahl der Prothesenmaterial-Elemente von 1 bis 30, vorzugsweise zwischen 2 und 15.

Durch die Anzahl der Taschenelemente wird dem Stentgraft die gewünschte Struktur und die gewünschten Eigenschaften gegeben. Durch das Vorhandensein mehrerer dicht hintereinander liegender Stent-Elemente ergibt sich beispielsweise eine weniger flexible Gefäßprothese, als eine mit weniger Stent-Elementen.

Für die Behandlung von besonders dünnwandigen bzw. empfindlichen Gefäßen kann es von Vorteil sein, dass innerhalb des Stentgrafts eine kontinuierliche Dichteveränderung der Stent-Elemente vorliegt. So kann es vorteilhaft sein, dass im proximalen Bereich des Stentgrafts besonders viele Stent-Elemente vorgesehen sind, wohingegen die Anzahl der Stent-Elemente in distale Richtung abnimmt. Hierdurch wird eine zunehmende Weichheit des Stentgrafts vom proximalen zum distalen Ende erreicht bzw. andersrum vom distalen Ende zum proximalen Ende eine kontinuierliche Zunahme der Steifheit des Stentgrafts.

Neben den Stent-Elementen ist es bevorzugt, dass auch der Durchmesser an das jeweilige zu behandelnde Gefäß angepasst wird. Der Stentgraft bzw. der Grundkörper des Stentgrafts kann so erfindungsgemäß über die gesamte Länge hinweg einen einheitlichen Durchmesser, oder aber unterschiedliche Durchmesser aufweisen. Der Durchmesser des Stentgrafts wird bestimmt über die Stent-Elemente und den hohlzylindrischen Grundkörper.

Gemäß einer Ausführungsform des erfindungsgemäßen Stentgrafts sind die Taschenelemente in einem Abstand x von 1 mm bis 30 mm, vorzugsweise zwischen 2 mm bis 15 mm, am Grundkörper fixiert.

Durch die Variabilität des Abstandes x von 1 mm bis 30 mm, vorzugsweise zwischen 2 mm bis 15 mm, kann der Stentgraft vorteilhaft an den jeweiligen Anwendungsbereich und somit an das entsprechende Gefäß angepasst werden. So kann sich aus einem sehr weiten Abstand der Taschenelemente ein flexiblerer Stentgraft ergeben als ein Stentgraft, bei dem die Taschenelemente in einem kleineren Abstand angebracht sind.

In einer Weiterbildung des erfindungsgemäßen Stentgrafts weist das Stent-Element eine einstückige Stentfeder auf oder besteht aus dieser, die abwechselnd zum proximalen und distalen Ende des Grundkörpers und parallel zu dessen Längsachse c weisenden Spitzbögen aufweist, wobei ein Spitzbogen aus jeweils einem Scheitelpunkt und zwei Schenkeln gebildet ist, wobei die Schenkel unterschiedliche oder gleiche Längen aufweisen.

Vorliegend wird unter einer "Stentfeder" jedes einstückige, ringförmige Element verstanden, dass sich aufgrund seines Materials komprimieren lässt und sich nach Entfernung des Kompressionsdrucks wieder federartig expandieren kann. Die Stentfeder weisen dabei einen wellenartigen Umlauf auf, wobei Wellenberg und Wellental eine Phase bilden und sich gegenseitig abwechseln.

Die Stent-Elemente bzw. Stentfedern können gleiche oder unterschiedliche umlaufende Amplituden aufweisen, welche sich aus den gleich langen bzw. unterschiedlich langen Schenkeln der Stentfedern ergeben. Unterschiedlich lange Amplituden, bieten den Vorteil, dass der Stentgraft an die jeweiligen Gefäße und die jeweiligen Beschaffenheit (Krümmungen, abgehende Gefäße, Verjüngungen, etc.) angepasst werden kann.

Die Stent-Elementen können erfindungsgemäß anstelle der einzelnen Stentfedern auch geflochtene, gezwirbelte oder Laser-geschnittene Stent-Elemente umfassen.

Erfindungsgemäß ist das Stent-Element innerhalb der Taschenelemente eingeschlossen und beweglich, mithin also nicht direkt und unmittelbar an dem ersten Prothesenmaterial durch Nähte oder anderweitig fixiert.

Gemäß einer weiteren Ausführungsform des erfindungsgemäßen Stentgrafts ist innerhalb eines Taschenelements ein einzelnes Stent-Element aufgenommen.

Aufgrund der Bauweise des erfindungsgemäßen Stentgrafts, sind die Taschenelemente so beschaffen, dass die Bewegungsfreiheit der Stent-Elemente innerhalb der Taschenelemente, aufgrund der Taschenelementgröße und der sich dadurch ergebene Spannung des diese bildenden Prothesenmaterials, stark eingeschränkt ist, bevorzugt bis zur Unbeweglichkeit der Stent-Elemente. Somit können die Stent-Elemente derartig vorteilhaft in den Taschenelemente eingesetzt werden, dass bei einer Krümmung des Stentgrafts keine unerwünschte Knicke oder Prothesenmaterial-Wülste bzw. Materialanhäufung auftreten.

Je nach Anwendungsbereich, und gemäß einer anderen Ausführungsform, können sich am Grundkörper auch einzelne Stent-Elemente nicht in Taschenelementen fixiert befinden, sondern zusätzlich am Grundkörper, beispielsweise über eine Naht innen oder außen fixiert sein. Dem Fachmann wird dabei klar sein, dass sich die Bauweise des Stentgrafts nach den Gegebenheiten der jeweiligen Gefäße richtet, die mit dem erfindungsgemäßen Stentgraft behandelt werden sollen.

Insbesondere ist bevorzugt, wenn jeweils am distalen und/oder proximalen Ende des Grundkörpers ein Stent-Elemente vorliegt, das direkt am ersten Prothesenmaterial des Grundkörpers - bspw. über Nähte - befestigt ist.

Gemäß einer weiteren Ausführungsform, ist ein am proximalen Ende des Stentgrafts angebrachtes Stent-Element nur über seine nach distal weisenden Spitzbögen, nicht aber seine nach proximal weisende Spitzbögen, am ersten Prothesenmaterial befestigt. Gemäß einer weiteren Ausführungsform ist ein am distalen Ende des Stentgrafts angebrachtes Stent-Element nur über seine nach proximal weisenden Spitzbögen am ersten Prothesenmaterial befestigt.

In einer bevorzugten Ausführungsform des Stentgrafts ist das Taschenelement am hohlzylindrischen Grundkörper angenäht. Beispielsweise durch eine umfängliche Naht.

Erfindungsgemäß begrenzt die umfängliche Naht die Tasche, in denen sich die Stent-Elemente befinden. Dabei ist die Naht bevorzugt so beschaffen, dass sich die Stent-Elemente in die geformten Taschen einsetzen lassen. Vorteilhafterweise kann die umfängliche Naht dabei beispielsweise maschinell angefertigt werden, sodass sich eine Zeit- und damit verbundener Kostenersparnis in der Herstellung des Stentgrafts ergibt. Zur Herstellung der Taschen wird zunächst ein Ende des Taschenelements umlaufend angenäht oder anderweitig am Grundköper fixiert. Anschließend wird das Stent-Element in die noch offene Tasche eingebracht und das Taschenelement durch Annähen oder anderweitigem Fixieren des zweiten Endes des Taschenelements verschlossen.

Bevorzugt wird als Nahtmaterial chirurgischer Faden verwendet. Dieser ist bevorzugt aus Polyester, Polyurethan, Polystyrol, Polytetrafluorethylen ultrahochmolekulargewichtiges Polyethylen (UHMPE), oder Mischungen davon ausgestaltet.

Neben der Fixierung des Taschenelements an den Grundkörpern durch eine Naht ist erfindungsgemäß auch eine Befestigung durch kleben oder verschmelzen bzw. schweißen möglich.

In einer weiteren bevorzugten Ausführungsform des Stentgrafts weisen das zumindest eine Taschenelement und das Stent-Element im Wesentlichen eine gleiche Höhe, bezogen auf die Längsachse c, auf.

Dabei bedeutet hier - wie auch überhaupt vorliegend - "im Wesentlichen", dass das Taschenelement und das Stent-Element nicht exakt die gleiche Höhe aufweisen, sondern dass auch eine annähernd gleiche Höhe möglich ist. Diese Ausführungsform hat den Vorteil, dass das Stent-Element durch die vorgegebene Taschen-Höhe in dieser annähernd bewegungsunfähig vorliegt. Damit fixiert die Tasche auf Grund ihrer Maße das Stent-Element.

In einer bevorzugten Ausführung des Stentgrafts weist dieser zumindest ein vom hohlzylindrischen Grundkörper nach innen oder außen abzweigenden Seitenast auf, der sich nach distal parallel zur Längsachse des hohlzylindrischen Grundkörpers erstreckt.

Mit "zumindest ein abzweigender Seitenast" ist/sind dabei vorliegend vorzugsweise ein, zwei, drei oder vier Seitenäste gemeint.

Mit dem Seitenast weist die Mantelfläche des Grundkörpers eine bzw. zumindest eine Öffnung auf, wodurch die Versorgung der abgehenden Seitengefäße über die Gefäßprothesen-Seitenäste sicher gewährleistet wird. Dies ist notwendig, bei der Anwendung des Stentgrafts innerhalb von Gefäßen, die Seitengefäße aufweisen. Dem Fachmann wird dabei klar sein, dass es auf die Gegebenheiten der jeweiligen Gefäße ankommt, die mit dem erfindungsgemäßen Stentgraft behandelt werden sollen.

Diese Ausführungsform bietet daher den Vorteil, dass dieser den jeweiligen anatomischen Verhältnissen des zu behandelnden Patienten angeglichen werden kann.

In einer bevorzugten Ausführung des Stentgrafts befindet sich auf dem Stentgraft ein Marker, der ein röntgendichtes Material enthält oder vollständig aus röntgendichtem Material besteht.

Mit Hilfe der Marker, die sich auf spezifischen Stellen des Stentgrafts befinden, ist es möglich besonders schnell die Lage des Stentgrafts während und nach der Implantation genau zu bestimmen.

Vorzugsweise sind die röntgendichten Marker aus einem oder mehreren der folgenden Materialien, bspw. Gold, Palladium, Tantal, Chrom, Silber, etc.; die Form der Marker kann dabei beliebig sein, bspw. rund eckig, und/oder bspw. die Form von Buchstaben, Zahlen oder Figuren haben, die für die Orientierung des Stentgrafts im Gefäß hilfreich sind.

In einer weiteren bevorzugten Ausführungsform des Stentgrafts weist das zumindest eine Stent-Element Nitinol auf oder ist aus diesem gebildet und kann von einem nicht expandierten Zustand in einen selbst-expandierten Zustand überführt werden.

Durch die Verwendung des selbstexpandierenden Material Nitinol weisen die Stent-Elemente Shape-Memory-Eigenschaften auf.

In einer bevorzugten Ausführung des Stentgrafts weist das erste und/oder das zweite Prothesenmaterial ein Material auf, dass ausgewählt ist aus einem Textil oder einem Polymer.

Insbesondere ist bevorzugt, wenn das erste und/oder zweite Prothesenmaterial ein Material aufweist oder aus diesem gebildet ist, das ausgewählt ist aus Polyester, Polyurethan, Polystyrol, Polytetrafluorethylen ultrahochmolekulargewichtiges Polyethylen (UHMPE), oder Mischungen davon.

In einer weiteren bevorzugten Ausführung ist das erste und zweite Prothesenmaterial aus demselben Material, oder weist zumindest teilweise unterschiedliche Materialien auf.

Gemäß einer weiteren Ausführungsform ist der erfindungsgemäße Stentgraft, wie weiter oben erwähnt, zur Behandlung von Dissektionserkrankungen oder aneurysmatischer Erkrankungen der Blutgefäße zu verwenden.

Ferner betrifft die vorliegende Erfindung auch ein Verfahren zur Freisetzung des erfindungsgemäßen Stentgrafts.

Weitere Vorteile ergeben sich aus den Figuren und der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
Fig. 1 eine schematische Darstellung einer Ausführungsform eines erfindungsgemäßen Stentgrafts, im nicht-eingeführten, expandierten Zustand in perspektivischer Ansicht der Längsseite von oben;
Fig. 2 ein Ausschnitt, der Ausführungsform aus Fig.1, wobei diese als Querschnitt entlang der Längsachse c von oben dargestellt ist;
Fig. 3 eine andere schematische Darstellung eines Ausschnitts der Ausführungsform eines erfindungsgemäßen Stentgrafts, in voller seitlicher Ansicht der Längsseite, in perspektivischer Ansicht der Längsseite von oben;
Fig. 4 eine schematische Darstellung eines Ausschnitts der Ausführungsform, eines erfindungsgemäßen Stentgrafts, welcher mit einem Seitenast versehen ist, dargestellt in perspektivischer Ansicht von oben; und
Fig. 5 eine weitere schematische Darstellung eines Ausschnitts der Ausführungsform eines erfindungsgemäßen Stentgrafts, wobei das proximalen Ende eine zum Teil freie Stentfeder aufweist, in perspektivischer Ansicht der Längsseite von seitlich oben.

In den Figuren werden gleiche Merkmale mit gleichen Bezugszeichen versehen, wobei aus Gründen der Übersichtlichkeit nicht in allen Figuren immer sämtliche Bezugszeichen angegeben sind.

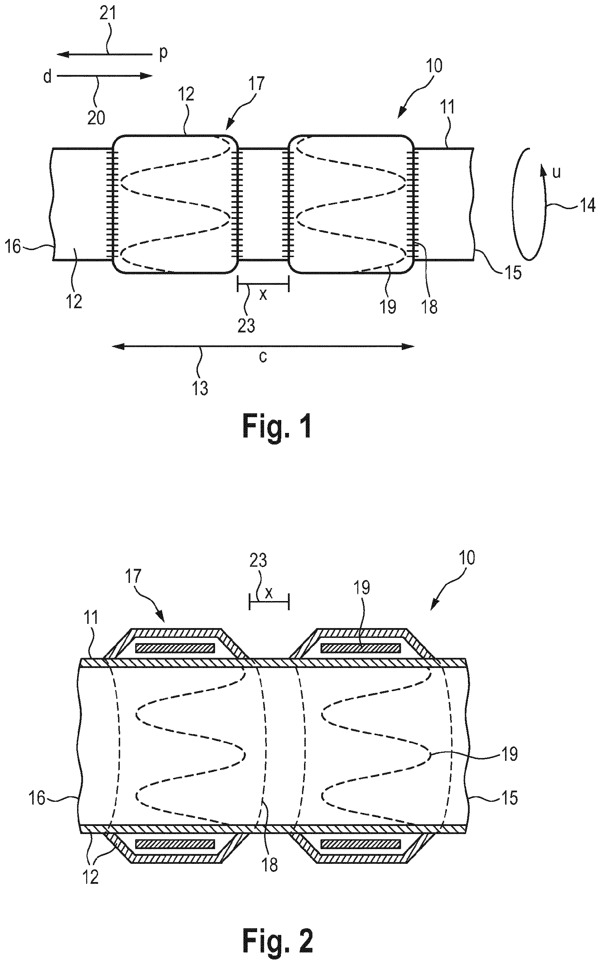

Wie aus den Figuren 1 bis 5 zu entnehmen ist, umfasst der selbstexpandierende Stentgraft 10 einen hohlzylindrischen Grundkörper 11 aus einem ersten Prothesenmaterial (Graft) 12 mit einer Längsachse c 13 und einem Umfang u 14. Der Stentgraft 10 weist zudem eine distale sowie proximale Öffnung 15, 16 auf. Der Grundkörper 11 bildet seinem Aufbau nach einen Mantel, an dem hohlzylindrische Taschenelemente 17 aus einem zweiten Prothesenmaterial 12 durch Nähte 18 angebracht sind. Dabei wird eine geschlossene Tasche 17 geformt, in die die Stent-Elemente 19 aufgenommen werden. Bei den Stent-Elementen 19 handelt es sich bevorzugt um Stentfedern. Neben den Stentfedern können auch geflochtene, gezwirbelte oder Laser-geschnittene Stent-Elemente 19 als Stent-Elemente 19 eingesetzt werden (nicht gezeigt), sofern diese selbstexpandierende Eigenschaften besitzen.

Die Struktur, bzw. die Beschaffenheit des Stentgrafts 10 wird maßgeblich durch die Struktur des Grundkörpers 11, als auch durch die umlaufenden Stent-Elemente 19 bestimmt. Während der Grundkörper 11 die Größe, bzw. die räumlichen Ausmaße vorgibt, bestimmen die Stent-Elemente 19 die selbstexpandierenden Eigenschaften als auch die Beschaffenheit (bspw. die Flexibilität) des Stentgrafts 10.

In den Figuren 1 und 3 bis 5 ist mit dem Pfeil "d" die distale Richtung 20 angezeigt, und mit dem Pfeil "p" die proximale Richtung 21. In Fig. 1 ist die Längsachse 13, die durch einen Doppelpfeil angezeigt ist, mit dem Buchstaben "c" bezeichnet. Der Umfang 14, der sich auf den Grundkörper 11 bezieht, ist mit dem Buchstaben "u" versehen.

Wie in allen Figuren ersichtlich ist, sind die Taschenelemente 17 vorzugsweise durch Nähte 18 am Grundkörper 11 befestigt. In einer nicht dargestellten Weise kann die Befestigung auch durch Kleben, Verschmelzen etc. erfolgen.

Die Taschenelemente 17 aus dem zweiten Prothesenmaterial 12 sind so beschaffen, dass die Stent-Elemente 19 optimal in deren Hohlraum passen, sodass sie auf Grund der Spannung des Prothesenmaterials 12 innerhalb der Taschen 17 fixiert und somit annähernd bewegungsunfähig sind.

Wie in den Figuren 1 bis 5 ersichtlich ist, bildet der Grundkörper 11 einen inneren Mantel. In einer alternativen Ausführungsform (nicht gezeigt) kann der Grundkörper 11 auch einen äußeren Mantel bilden, in dessen Innenraum die taschenformenden Taschenelemente 17 befestigt sind. In einer weiteren alternativen Ausführungsform (nicht gezeigt) können sich die Taschen 17 sowohl innen als auch außenliegend, in Bezug zum Grundkörper 11, befinden.

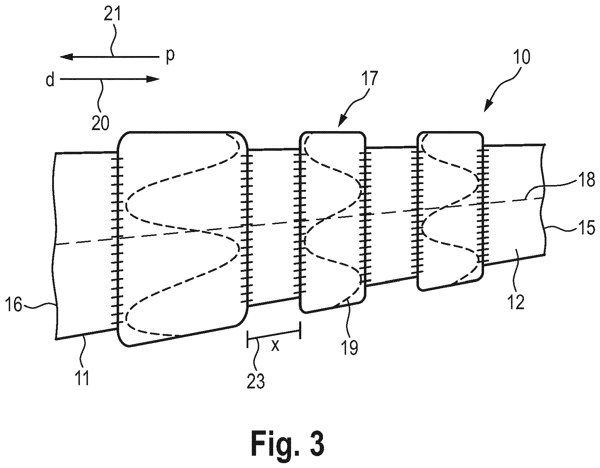

Bei den Stent-Elementen 19 handelt es sich vorzugsweise um Stentfedern, die mäanderförmig den Grundkörper 11 umlaufen. Wie Figur 3 bis 5 zu entnehmen ist, sind die Stentfedern nicht zwingend identisch in ihrer Größe und Form.

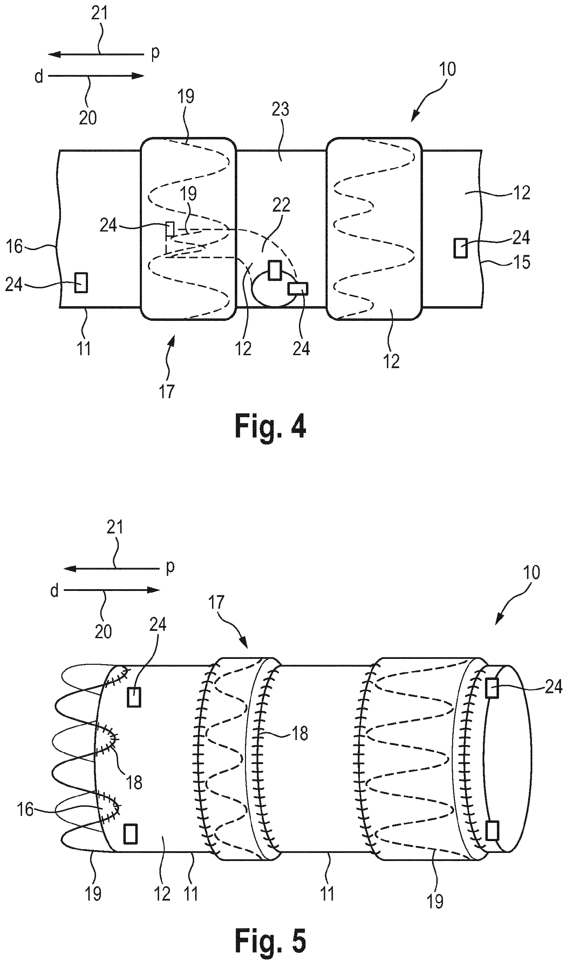

Die Stentfedern liegen erfindungsgemäß innerhalb der Taschen 17 vor. Alternativ oder zusätzlich können einzelne Stentfedern auch direkt innerhalb oder außerhalb am Grundkörper 11 befestigt werden, vorzugsweise mit Hilfe einer Naht 18, wie auch aus der Figur 5 ersichtlich ist.

Im Wesentlichen weist der Stentgraft 10 entlang der Längsachse 13 einen konstanten Durchmesser auf. Dies ist jedoch abhängig vom jeweiligen zu behandelnden Blutgefäß, in welches der Stentgraft 10 implantiert werden soll. Wie insbesondere aus Fig. 3 zu entnehmen ist, kann das proximale Ende 16 gegenüber dem distalen Ende 15 einen größeren Umfang 14 aufweisen. Somit ergibt sich eine kontinuierliche Verjüngung in distale Richtung 20. Der sich entlang der Längsachse 13 ergebende unterschiedliche Umfang 14, wird zum einen durch den Grundkörper 11 an sich, als auch durch die umgebenen Stentfedern erreicht. So weisen die Stentfedern in proximale Richtung 21 einen größeren Durchmesser auf, als die in distaler Richtung 20. Eine kontinuierliche Veränderung des Umfangs 14 ergibt sich bevorzugt, sowohl aus den Stentfedern als auch aus dem Grundkörper 11. Hierbei soll eine Materialanhäufung, sowie Prothesenmaterialwülste vermieden bzw. verhindert werden.

Fig.3 zeigt, dass durch eine Naht 18 entlang der Längsachse c 13 der Grundkörper 11 seine hohlzylindrische Form erhält. Eine Naht 18 ist nicht zwingend notwendig, alternativ kann der Grundkörper 11 bereits aus einem hohlzylindrischen Prothesenmaterial 12 bestehen.

Fig.4 ist ferner zu entnehmen, dass der Stentgraft 10 einen Seitenast 22 umfasst. Dieser ist so angebracht, dass er sich nach innen, ins Innere, d.h. also ins Lumen des Grundkörpers 11, erstreckt. Somit besitzt der Seitenast 22 eine erste Öffnung, welche fest im Prothesenmaterial 12 des Grundkörpers 11 (innerhalb des Abschnitts x ) angebracht ist und eine zweite Öffnung, die sich ins Lumen des Grundkörpers 11 erstreckt.

Der Seitenast 22 weist ein Stent-Element 19 auf, das sich im Bereich der zweiten Öffnung befindet, wodurch diese Öffnung offen gehalten wird. Das Stent-Element 19 bildet zusammen mit dem Prothesenmaterial 12 den Seitenast 22. Bei dem Seitenast 22 handelt es sich folglich um einen weiteren kleineren Stentgraft, welcher an dem eigentlichen Stentgraft 10 fixiert ist.

Das Stent-Element, welches mäanderförmig den Seitenast 22 umläuft, kann durch Nähte 18 direkt am Prothesenmaterial 12 befestigt sein, oder sich in Taschen 17 aus Prothesenmaterial 12 befinden, analog zu den Stent-Elementen 19, welche um den Grundkörper 11 befestigt sind.

Die Größe der Seitenäste 22 wird an die jeweils zu behandelnden Blutgefäß angepasst. Der Stentgraft 10 ist erfindungsgemäß nicht auf einen Seitenast 22 beschränkt, vielmehr richtet sich die Anzahl der Seitenäste 22 nach den Gegebenheiten der jeweils im Patienten vorliegenden Gefäße.

Wie aus den Figuren 4 und 5 ersichtlich ist, weist der Stentgraft 10 an spezifischen Stellen Röntgenmarker 24 auf. So befinden sich die röntgendichten Marker 24 zum einen am distalen Ende 15 als auch rund um die Öffnung des Seitenasts 22. Die Marker 24 sind notwendig, um die richtige Positionierung innerhalb des Gefäßes während der Implantation zu gewährleisten. Der behandelnde Arzt kann so die genaue Position des Stentgrafts 10 innerhalb des Gefäßes in der Röntgenkontrolle schnell und genau bestimmen und gegebenenfalls während der Implantation korrigieren.

Zum einen kann der Grundkörper 11, wie in Fig. 1 gezeigt, die proximalen bzw. distalen Enden des Stentgrafts 10 bilden. Zum anderen kann ein Ende des Stentgrafts 10 auch mit einem Taschenelement 17 enden (nicht gezeigt). Eine weitere mögliche Ausgestaltung des proximalen Endes des Stentgrafts 10 ist in Fig. 5 gezeigt, in welcher eine Stentfeder am proximalen Ende 16 nur zum Teil am Grundkörper 11 des Prothesenmaterials 12 angebracht ist, bevorzugt mit Hilfe einer Naht 18.

Zur Einführung des erfindungsgemäßen Stentgraft 10 wird der Stentgraft 10 auf ein Einführsystem geladen (nicht gezeigt) und über eine entsprechende Hülle (nicht gezeigt) in einen komprimierten Zustand gehalten. Verfahren und Vorrichtungen zum Einführen von Stentgrafts 10 sind dem Fachmann aus dem Stand der Technik geläufig.

Zunächst wird der komprimiert gehaltene Stentgraft 10 in das zu behandelnde Gefäß vorgeschoben. Die korrekte Platzierung kann bspw. über entsprechende, an dem Stentgraft 10 vorgesehene - bspw. röntgendichte - Marker 24 kontrolliert werden. Nach korrekter Platzierung kann der Stentgraft 10 nun durch Rückziehen einer den Stentgraft 10 komprimiert haltenden Hülle o.ä. freigesetzt werden.

## Patentansprüche

1. Stentgraft (10) zur Implantation in Gefäße eines Patienten, wobei der Stentgraft (10) folgendes aufweist:
einen aus einem ersten Prothesenmaterial (12) bestehenden hohlzylindrischen Grundkörper (11), mit einem proximalen und einem distalen Ende, mit einer Längsachse c (13) und einem Umfang u (14),
zumindest ein aus einem zweiten Prothesenmaterial (12) bestehendes Taschenelement (17), das umlaufend auf der Außen- und/oder Innenseite des Grundkörpers (11) zur Ausbildung einer umlaufenden geschlossenen Tasche (17) auf einem Längsabschnitt des Grundkörpers (11) angebracht ist, und
mindestens ein den Grundkörper (11) mäanderförmig umlaufendes Stent-Element (19), das innerhalb des Taschenelements (17) aufgenommen ist,
wobei das zumindest eine Taschenelement (17) kürzer als der Grundkörper (11) ist, und ein proximales und ein distales Ende sowie einen dazwischen liegenden Hauptabschnitt aufweist, wobei das zumindest eine Taschenelement (17) zur Ausbildung der geschlossenen Tasche (17) lediglich über sein jeweils distales und proximales Ende umfänglich außen und/oder innen am Grundkörper (11) befestigt und der Hauptabschnitt nicht am Grundkörper (11) befestigt ist, **dadurch gekennzeichnet, dass** 2, 3, 4, 5, 6, 7, 8, 9, 10, 11, 12, 13, 14, 15, 16, 17, 18, 19, 20, 25 oder 30 Taschenelemente (17) vorgesehen sind, die in einem Abstand x und getrennt voneinander auf dem Grundkörper (11) fixiert sind.

2. Stentgraft (10) nach Anspruch 1, **dadurch gekennzeichnet, dass** die Taschenelemente (17) in einem Abstand 1 mm bis 30 mm am Grundkörper (11) fixiert sind.

3. Stentgraft (10) nach einem der Ansprüche 1 oder 2, **dadurch gekennzeichnet, dass** das Stent-Element (19) eine einstückige Stentfeder aufweist oder aus dieser besteht, die abwechselnd zum proximalen und distalen Ende des Grundkörpers (11) und parallel zu dessen Längsachse c (13) weisenden Spitzbögen aufweisen, wobei ein Spitzbogen aus jeweils einem Scheitelpunkt und zwei Schenkeln gebildet ist, wobei die Schenkel unterschiedliche oder gleiche Längen aufweisen.

4. Stentgraft (10) nach einem der Ansprüche 1 bis 3, **dadurch gekennzeichnet, dass** innerhalb eines Taschenelements (17) ein einzelnes Stent-Element (19) aufgenommen ist.

5. Stentgraft (10) nach einem der Ansprüche 1 bis 4, **dadurch gekennzeichnet, dass** das Taschenelement (17) am hohlzylindrischen Grundköper (11) angenäht ist.

6. Stentgraft (10) nach einem der Ansprüche 1 bis 5, **dadurch gekennzeichnet, dass** das Taschenelement (17) und das Stent-Element (19) eine im Wesentlichen gleiche Höhe, bezogen auf die Längsachse c (13), aufweisen.

7. Stentgraft (10) nach einem der Ansprüche 1 bis 6, **dadurch gekennzeichnet, dass** dieser zumindest ein vom hohlzylindrischen Grundkörper (11) nach innen oder au-ßen abzweigenden Seitenast (22) aufweist, der sich nach distal parallel zur Längsachse (13) des hohlzylindrischen Grundkörpers (11) erstreckt.

8. Stentgraft (10) nach einem der Ansprüche 1 bis 7, **dadurch gekennzeichnet, dass** sich auf dem Stentgraft (10) ein Marker (24) befindet, der ein röntgendichtes Material enthält oder vollständig aus röntgendichtem Material besteht.

9. Stentgraft (10) nach einem der Ansprüche 1 bis 8, **dadurch gekennzeichnet, dass** das zumindest eine Stent-Element (19) Nitinol aufweist oder aus diesem gebildet ist, und von einem nicht expandierten Zustand in einen selbst-expandierten Zustand überführt werden kann.

10. Stentgraft (10) nach einem der Ansprüche 1 bis 9, **dadurch gekennzeichnet, dass** das erste und/oder zweite Prothesenmaterial (12) ein Material aufweist, dass ausgewählt ist aus einem Textil oder einem Polymer.

11. Stentgraft (10) nach einem der Ansprüche 1 bis 10, **dadurch gekennzeichnet, dass** das erste und/oder zweite Prothesenmaterial (12) ein Material aufweist oder aus diesem gebildet ist, das ausgewählt ist aus Polyester, Polyurethan, Polystyrol, Polytetrafluorethylen, ultrahochmolekulargewichtiges Polyethylen (UHMPE), oder Mischungen davon.

12. Stentgraft (10) nach einem der Ansprüche 1 bis 11, **dadurch gekennzeichnet, dass** das erste und zweite Prothesenmaterial (12) aus demselben Material oder zumindest teilweise unterschiedlich Materialien aufweisen.

## Claims

1. A stent graft (10) for implantation in vessels of a patient, wherein the stent graft (10) has the following:
a hollow-cylindrical body (11) made of a first prosthesis material (12), with a proximal end and a distal end, and with a longitudinal axis c (13) and a circumference u (14),
at least one pocket element (17), which is made of a second prosthesis material (12) and which is mounted circumferentially on the outer and/or inner face of the body (11) in order to form a circumferential closed pocket (17) on a longitudinal portion of the body (11), and
at least one stent element (19), which extends in a meandering formation around the body (11) and is received inside the pocket element (17), wherein the at least one pocket element (17) is shorter than the body (11) and has a proximal end and a distal end and, positioned between these, a main portion (23),
wherein the at least one pocket element (17) for forming the closed pocket (17) is circumferentially secured only via its respective distal and proximal end to the outside and/or inside of the body (11), and the main portion (23) is not secured to the body (11),
**characterized in that** 2,3,4,5,6,7,8,9,10,11,12,13,14,15,16,17,18,19,20, 25 or 30 pocket elements (17) are provided, which are fixed at a distance from each other and separate from each other on the body (11).

2. The stent graft (10) as claimed in claim 1, **characterized in that** the pocket elements (17) are fixed at a distance of 1 mm to 30 mm on the body (11).

3. The stent graft (10) as claimed in one of claims 1 or 2, **characterized in that** the stent element (19) has or consists of a one-piece stent spring, with pointed arches facing alternately to the proximal and distal end of the body (11) and parallel to the longitudinal axis c (13) of the latter, wherein a pointed arch is formed in each case from a vertex and two legs, wherein the legs have different or identical lengths.

4. The stent graft (10) as claimed in one of claims 1 through 3, **characterized in that** an individual stent element (19) is received inside a pocket element (17).

5. The stent graft (10) as claimed in one of claims 1 through 4, **characterized in that** the pocket element (17) is sewn onto the hollow-cylindrical body (11).

6. The stent graft (10) as claimed in one of claims 1 through 5, **characterized in that** the pocket element (17) and the stent element (19) have substantially the same height, relative to the longitudinal axis c (13).

7. The stent graft (10) as claimed in one of claims 1 through 6, **characterized in that** it has at least one side branch (22) branching off inwardly or outwardly from the hollow-cylindrical body (11) and extending in the distal direction parallel to the longitudinal axis (13) of the hollow-cylindrical body (11).

8. The stent graft (10) as claimed in one of claims 1 through 7, **characterized in that** a marker (24) is located on the stent graft (10), which marker (24) contains a radiopaque material or is made entirely of radiopaque material.

9. The stent graft (10) as claimed in one of claims 1 through 8, **characterized in that** the at least one stent element (19) comprises or is formed from nitinol and can be converted from a non-expanded state to a self-expanded state.

10. The stent graft (10) as claimed in one of claims 1 through 9, **characterized in that** the first and/or second prosthesis material (12) comprises a material that is chosen from a textile or a polymer.

11. The stent graft (10) as claimed in one of claims 1 through 10, **characterized in that** the first and/or second prosthesis material (12) comprises or is formed from a material that is chosen from polyester, polyurethane, polystyrene, polytetrafluoroethylene, ultra-high molecular weight polyethylene (UHMPE), or mixtures thereof.

12. The stent graft (10) as claimed in one of claims 1 through 11, **characterized in that** the first and second prosthesis materials (12) are the same material or at least partially different materials.

## Revendications

1. Endoprothèse (10) destinée à être implantée dans les vaisseaux d'un patient, dans laquelle l'endoprothèse (10) comprend les éléments suivants :
un corps de base (11) cylindrique creux constitué d'un premier matériau de prothèse (12), comportant une extrémité proximale et une extrémité distale, comportant un axe longitudinal c (13) et une circonférence u (14),
au moins un élément formant poche (17) constitué d'un second matériau de prothèse (12) et monté sur le pourtour sur la face extérieure et/ou la face intérieure du corps de base (11) pour former une poche (17) fermée sur le pourtour sur une section longitudinale du corps de base (11), et
au moins un élément formant extenseur (19) entourant le corps de base (11) en forme de méandres et logé à l'intérieur de l'élément formant poche (17),
dans laquelle l'au moins un élément formant poche (17) est plus court que le corps de base (11) et présente une extrémité proximale et une extrémité distale ainsi qu'une section principale située entre celles-ci, dans laquelle, pour former la poche (17) fermée, l'au moins un élément formant poche (17) est fixé au corps de base (11) uniquement par l'intermédiaire de son extrémité distale et de son extrémité proximale respectives, sur la circonférence à l'extérieur et/ou à l'intérieur, et la section principale n'est pas fixée au corps de base (11),
**caractérisée en ce que** 2, 3, 4, 5, 6, 7, 8, 9, 10, 11, 12, 13, 14, 15, 16, 17, 18, 19, 20, 25 ou 30 éléments formant poches (17) sont prévus, lesquels sont fixés à une distance x et séparément les uns des autres sur le corps de base (11).

2. Endoprothèse (10) selon la revendication 1, **caractérisée en ce que** les éléments formant poches (17) sont fixés au corps de base (11) à une distance allant de 1 mm à 30 mm.

3. Endoprothèse (10) selon l'une des revendications 1 ou 2,
**caractérisée en ce que** l'élément formant extenseur (19) présente ou est constitué d'un ressort d'extenseur d'une seule pièce qui présente des ogives orientées alternativement vers l'extrémité proximale et vers l'extrémité distale du corps de base (11) et parallèlement à son axe longitudinal c (13), dans laquelle une ogive est formée respectivement d'un sommet et de deux bras, dans laquelle les bras présentent des longueurs différentes ou égales.

4. Endoprothèse (10) selon l'une des revendications 1 à 3, **caractérisée en ce qu'**un seul élément formant extenseur (19) est logé à l'intérieur d'un élément formant poche (17).

5. Endoprothèse (10) selon l'une des revendications 1 à 4,
**caractérisée en ce que** l'élément formant poche (17) est cousu au corps de base (11) cylindrique creux.

6. Endoprothèse (10) selon l'une des revendications 1 à 5,
**caractérisée en ce que** l'élément formant poche (17) et l'élément formant extenseur (19) présentent une hauteur sensiblement identique, par rapport à l'axe longitudinal c (13).

7. Endoprothèse (10) selon l'une des revendications 1 à 6,
**caractérisée en ce que** celle-ci présente au moins une branche latérale (22) partant du corps de base (11) cylindrique creux vers l'intérieur ou l'extérieur et s'étendant vers le côté distal parallèlement à l'axe longitudinal (13) du corps de base (11) cylindrique creux.

8. Endoprothèse (10) selon l'une des revendications 1 à 7, **caractérisée en ce qu'**un marqueur (24) se trouve sur l'endoprothèse (10), lequel contient un matériau radio-opaque ou est entièrement constitué d'un matériau radio-opaque.

9. Endoprothèse (10) selon l'une des revendications 1 à 8,
**caractérisée en ce que** l'au moins un élément formant extenseur (19) présente du nitinol ou est formé à partir de celui-ci, et peut être transféré d'un état non expansé à un état auto-expansé.

10. Endoprothèse (10) selon l'une des revendications 1 à 9,
**caractérisée en ce que** le premier et/ou le second matériau de prothèse (12) présentent un matériau qui est choisi parmi un textile ou un polymère.

11. Endoprothèse (10) selon l'une des revendications 1 à 10,
**caractérisée en ce que** le premier et/ou le second matériau de prothèse (12) présentent ou sont formés à partir d'un matériau qui est choisi parmi polyester, polyuréthane, polystyrène, polytétrafluoroéthylène, polyéthylène à poids moléculaire ultra-élevé (UHMPE), ou des mélanges de ceux-ci.

12. Endoprothèse (10) selon l'une des revendications 1 à 11,
**caractérisée en ce que** les premier et second matériaux de prothèse (12) présentent le même matériau ou des matériaux au moins partiellement différents.
